(19) Europäisches Patentamt
European Patent Office
Office européen des brevets

(11) **EP 3 513 837 A1**

(12) **EUROPEAN PATENT APPLICATION**
published in accordance with Art. 153(4) EPC

(43) Date of publication:
**24.07.2019 Bulletin 2019/30**

(51) Int Cl.:
**A61N 5/10** *(2006.01)*          **A61L 31/06** *(2006.01)*

(21) Application number: **17850960.0**

(86) International application number:
**PCT/JP2017/033200**

(22) Date of filing: **14.09.2017**

(87) International publication number:
**WO 2018/052059 (22.03.2018 Gazette 2018/12)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **15.09.2016 JP 2016181028**

(71) Applicants:
• **National University Corporation Kobe University
Kobe-shi, Hyogo 657-8501 (JP)**
• **Kanai Juyo Kogyo Co., Ltd.
Itami-shi, Hiyogo 664-0025 (JP)**
• **Alfresa Pharma Corporation
Osaka-shi, Osaka 540-8575 (JP)**

(72) Inventors:
• **SASAKI, Ryohei
Kobe-shi
Hyogo 657-8501 (JP)**

• **FUKUMOTO, Takumi
Kobe-shi
Hyogo 657-8501 (JP)**
• **INUBUSHI, Sachiko
Kobe-shi
Hyogo 657-8501 (JP)**
• **OBATA, Tsutomu
Itami-shi
Hyogo 664-0025 (JP)**
• **TAGAMI, Yoshitaka
Itami-shi
Hyogo 664-0025 (JP)**

(74) Representative: **Isarpatent
Patent- und Rechtsanwälte Behnisch Barth
Charles
Hassa Peckmann & Partner mbB
Friedrichstrasse 31
80801 München (DE)**

(54) **SPACER FOR RADIATION THERAPY**

(57)     Disclosed is a spacer for radiotherapy that can be easily compressed into a reduced thickness, and has a high capacity to reconstitute itself from a compressed state back to its initial thickness after removal of external force. The spacer for radiotherapy is a flexible structure having front and back main faces and thickness therebetween, and composed of strands of biocompatible and biodegradable synthetic fibers, wherein the spacer consists of (a) a pair of main face portions opposing each other with a gap therebetween and respectively defining the main faces of the structure, and (b) a linker portion linking between the pair of main face portions, (c) wherein the main face portions comprise a knitted-fabric structure or a weave structure made of the strands, and (d) wherein the linker portion is composed as a collection of linker strands formed of the said strands and respectively extending in the thickness direction of the spacer and linking the pair of main face portions with each other.

EP 3 513 837 A1

Fig. 1

**Description**

TECHNICAL FIELD

The present invention relates to therapeutic treatment of malignant tumors such as cancers including sarcomas, in various kinds of radiation therapies like particle radiation therapy and interstitial radiation therapy, more specifically to a spacer which is inserted between an affected site and surrounding normal tissues to protect those normal tissues from radiation exposure during a radiotherapy of a malignant tumor, and in particular, to such a spacer suitable to be introduced into the affected site utilizing endoscopic surgery performed with laparoscope or the like.

BACKGROUND ART

The incidence of malignant tumors correlates to ages, and in Japan, for example, more than half of annual deaths caused by malignant tumors take place in aged people of 75 years or above. As it is often difficult in aged people to perform a radical surgery in an open abdominal procedure, which is highly invasive, to treat such cancers as pancreatic, liver, and bile duct cancers, there is a need for development of a less invasive, yet radical treatment that is comparable to surgery.

While radiotherapy, including particle radiation therapy and interstitial radiation therapy, is a low invasive treatment of malignant tumors, it is difficult to irradiate the tumor with doses adopted for radical therapy when considering risks of radiation damage to normal tissues adjacent to the tumor to be irradiated. Consequently, a kind of therapy, is becoming popular as a combination of surgical and radiation therapies, in which tumors are irradiated with doses adapted for radical treatment, while protecting normal tissues from radiation exposure, by surgically placing a spacer to gain a treatment space and also to keep intervals between the tumor and adjacent normal tissues.

At present, a spacer for radiotherapy has not been on the market yet, either in or out of Japan. Thus, to address this difficulty, certain conventional medical devices are employed at medical front as alternatives to a pacer, such as artificial blood vessel, pericardium sheet, tissue expander (silicone bag), collagen sponge, and the like. However, those medical devices pose such problems that they are not absorbable by the body but must be taken out by additional surgery which is not risk-free (e.g., silicone bag), or that some of them originating from animals entails a risk of viral infection (e.g., collagen sponge). Furthermore, since they are not intended for use as a spacer, they have additional drawbacks such as poor radiation shielding capacity, and difficulty in and costliness of processing them into a spacer.

In order to solve the above problems, radiotherapy spacers have been proposed that comprise a fiber assembly formed of three-dimensionally entangled fibers of biocompatible synthetic polymers (Patent Documents 1 and 2). Those radiotherapy spacers exhibit advantageous effects in that they offer protection of surrounding normal tissues by effectively shielding them from radiation utilizing the water held by the fiber assembly, and also in that additional surgery for removing them is not required if they are made of a bioabsorbable material.

Meanwhile, laparoscopic surgery has been actively adopted in recent years as a surgical procedure that can lessen the physical burden on patients having malignant tumor to be receiving surgery. Laparoscopic surgery is a type of surgery performed by making a few small incisions on the surface of patient's abdomen, inserting trocars (trocar tube) having a hollow cylindrical insertion part into the abdominal cavity of the patient through the incisions, inserting instruments for laparoscopic surgery with attached devices such as a CCD camera through the lumen of the trocars, and manipulating those instruments outside the body. Because of its advantages over conventional open surgery such as (1) less pain after surgery and less noticeable operative scar, (2) reduced bleeding, and (3) possibility of earlier leaving the hospital, laparoscopic surgery is significantly needed by patients.

However, if a spacer disclosed in Patent Document 1 or 2 is to be placed in a patient who needs radiotherapy, open surgery is inevitable. Namely, the spacers specifically described as preferable in Patent Documents 1 and 2 are made of non-woven fabric, and particularly those with their vertical and horizontal dimensions of several centimeters. While this description reflects the general necessity of such sizes for them to function as spacers when inserted between tissues or organs, such sizes go far beyond the internal diameter of a trocar, which is around 10 mm. Furthermore, non-woven-fabric spacers of such sizes can never be compressed and deformed into sizes that would allow them to be passed through the internal diameter of a trocar, by limited amount of force applicable with a hand. Thus, those non-woven-fabric spacers cannot be introduced into the abdominal cavity through the lumen of a trocar, and therefore open surgery is inevitable.

PRIOR ART DOCUMENTS

PATENT DOCUMENTS

[Patent Document 1] WO 2011/055670
[Patent Document 2] WO 2015/098904

SUMMARY OF INVENTION

TECHNICAL PROBLEM

Thus, against the abovementioned background, there are potential needs of a radiotherapy spacer that can be significantly compressed and deformed, by a level of force applicable with a hand, so that it can be introduced into the body through a trocar, and also has a capacity to reconstitute itself, after such deformation, almost back to the initial state. Such a spacer might be used in radiotherapy without performing open surgery of a patient, for it could be put into the lumen of a trocar in a compressed and deformed state, and once having introduced through the trocar into the body, reconstitute itself from the compressed state back into its initial state.

Focusing on the above potential needs, it is the objective of present invention to provide a spacer for radiotherapy that can be easily compressed into a far reduced thickness compared with its initial state by a small amount of external force readily applicable with a hand (easy compressibility), and has a high capacity to reconstitute itself from such a compressed state back into its initial thickness after removal of such an external force (high reconstitutability).

SOLUTION TO PROBLEM

In a study with the above objective, the present inventors found that a structure made of fibers organized into a certain structure possesses both easy compressibility and high reconstitutability. The present invention was completed through further studies based on this finding. Thus, the present invention provides what follows.

1. A spacer for radiotherapy that is a flexible structure having front and back main faces and thickness therebetween, and composed of strands of biocompatible and biodegradable synthetic fibers,
wherein the spacer consists of

(a) a pair of main face portions opposing each other with a gap therebetween and respectively defining the main faces of the structure, and
(b) a linker portion linking between the pair of main face portions ,
and
(c) wherein the main face portions comprise a knitted-fabric structure or a weave structure made of the strands, and
(d) wherein the linker portion is composed as a collection of linker strands formed of the said strands respectively extending in the thickness direction of the spacer and linking the pair of main face portions with each other.

2. The spacer for radiotherapy according to 1 above possessing a rate of compressibility of at least 70% and a rate of compression resilience of at least 80%, as determined in accordance with JIS L 1913 (Test Methods for Nonwovens).
3. The spacer for radiotherapy according to 1 or 2 above having a density as a whole of 50-200 mg/cm$^3$.
4. The spacer for radiotherapy according to one of 1-3 above, wherein the density of the linker portion is at least 30

EFFECTS OF INVENTION

The spacer for radiotherapy according to the present invention possesses easy compressibility and high reconstitutability, simultaneously. Therefore, the spacer can be made thinner easily by pressing it with fingers, and be passed through the lumen of a trocar in a compact state with its volume greatly reduced by any desired method, such as sequentially compressing its thickness starting from one of its edges while rolling it up into a cylindrical form, and after having passed out of the lumen of the trocar, it is allowed to expand almost back to its initial state. Thus, according to the present invention, although being a spacer for radiotherapy, yet it can be placed in the body following a less invasive procedure of laparoscopic surgery while avoiding an open surgery. Furthermore, the present invention enables provision of such spacers exhibiting excellent uniformity of performances that is almost free of fluctuation both in its easy compressibility and high reconstitutability.

BRIEF DESCRIPTION OF DRAWINGS

Fig. 1 is a schematic illustration of the general appearance of an example of the radiotherapy spacer according to the present invention.

Fig. 2 is a schematic illustration of an enlarged view of part of a cross section of the linking portion of another example of the radiotherapy spacer according to the present invention.

Fig. 3 is a schematic illustration of an enlarged view of part of a cross section of the linking portion of still another example of the radiotherapy spacer according to the present invention.

Fig. 4 is a schematic illustration of an enlarged view of part of a cross section of the linking portion of still another example of the radiotherapy spacer according to the present invention.

Fig. 5 shows photographs of an example of the radiotherapy spacer (a) in its expanded state before compression, and (b) in its state having been rolled-up into a cylindrical form under simultaneous compressing starting from an edge onwards, respectively.

Fig. 6 is a graph illustrating the rate of compressibility and the rate of compression resilience of an example of the radiotherapy spacer in comparison with those of a spacer made of non-woven fabric.

DESCRIPTION OF EMBODIMENTS

The radiotherapy spacer according to the present invention is composed of strands of biocompatible and biodegradable synthetic fibers, and is a flexible structure having front and back main faces and thickness between them. The pair of layer-like main face portions, front and back (besides, there has to be no distinction between front and back faces), are composed in a layer-like form of knitted or woven strands of biocompatible and biodegradable synthetic fibers, and are themselves flexible. Although it is also composed of biocompatible and biodegradable synthetic fibers as the main face portions, the linker portion, which provides the radiotherapy spacer with its thickness, has a different morphology from that of the main face portions. Namely, the linker portion is composed as a dense collection of linker strands that extend so as to bridging the pair of main face portions (their inner face) and link them.

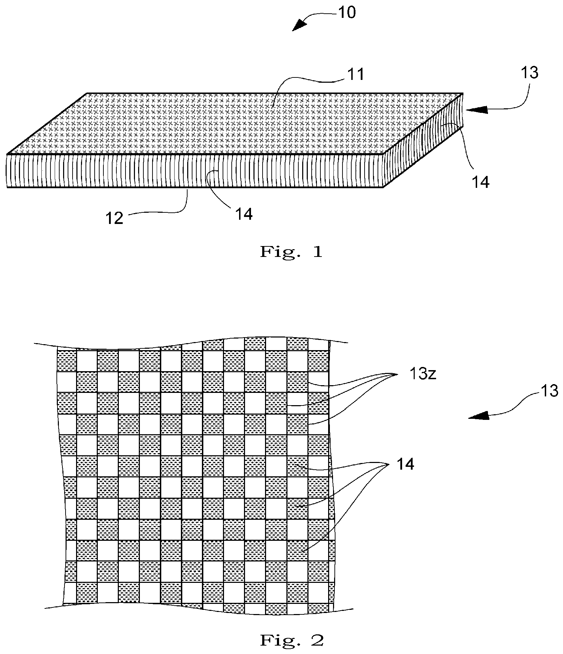

The linker strands composing the linker portion may be arranged in such a manner that each of them extends in almost the same direction as that of adjacent linker strands (generally parallel), as shown in Fig. 1, or arranged varying the direction in which the linker strands extend so that some of the linker strands cross one another. From the viewpoint of easy compressibility, it is preferable that linker strands are arranged almost parallel so that they can fall together in the same direction. On the other hand, in point of a spacer easily keeping its thickness, escaping collapse even when loaded with a substantial amount of multiangle pressures between organs, it is expected to be preferable that some of linker strands are arranged to cross with one another.

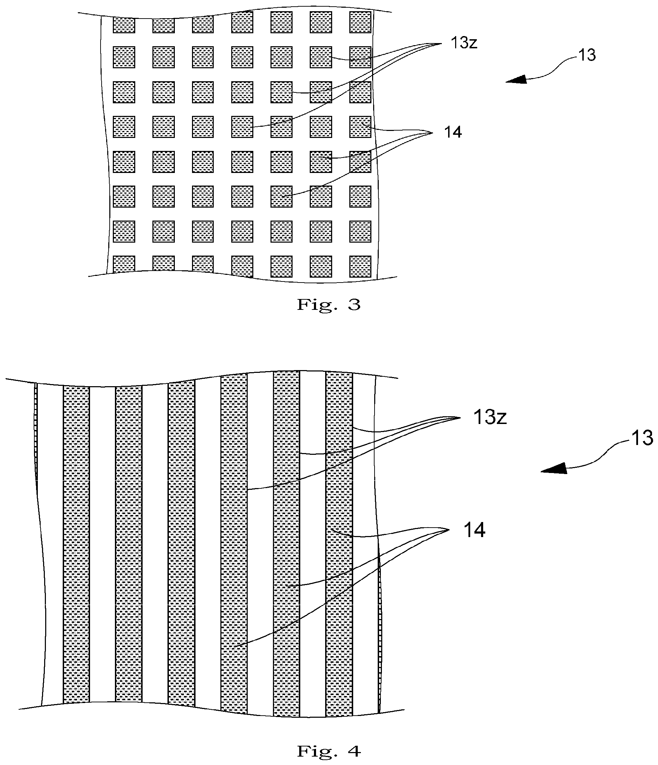

Besides, the linker portion does not necessarily take the form that linker strands (14) are evenly distributed across the entire area of the main face portions such as the one shown in Fig. 1. Namely, insofar as it allows the front (11) and back (12) face portions of a spacer (10) to be handled as a single body, the linker portion (13) may be built as a collection of distinct, mutually separated linker-strands-distributed zones (13z) (linker strands (14) are densely arranged in those zones). Fig.2 illustrates an example of the collection of multiple linker-strand-distributed zones (13z), where those zones are in contact with each other only partially and as a whole, mutually separated in two directions (horizontally and vertically, in the figure); Fig. 3 illustrates an example of the collection of multiple linker-strand-distributed zones (13z), where the zones are mutually separated in two directions; and Fig. 4 illustrates an example of the collection of multiple (band like) linker-strand-distributed zones (13z), where the zones are separated in only one direction. In the case where a linker portion (13) is built as a collection of multiple, mutually separated linker-strand-distributed zones (13z), the linker strands (14) are more easily made to fall down, leading to easy compressibility of the thickness. Besides, though Figs. 2-4 set forth examples in which the shape of each linker-strand-distributed zone (13z) is a square or rectangular in plan view, a linker portion (13) is not restricted to such particular shapes, for it may be designed to be a shape taking account of a balance between easy compressibility and high reconstitutability. Feasible shapes in plan view of each linker-strand-distributed zone (13z) may be pentagon-like, e.g., trianglular, square, rectangular, rhombic, and pentagonal; circular or semicircular, or also a form produced by their combination. Further, a linker portion may also be composed as a mixture of linker-strand-distributed zones (13z) having different shapes in plan view.

The structure as mentioned above may be produced by well known techniques in the field of textiles and clothing in general, such as Double Raschel knitting, using biocompatible and biodegradable continuous fibers and an apparatus, such as Double-Bar Raschel machine by Karl Myer. Alternatively, the structure can also be produced as an uncut velvet in a production process of velvet, which is a woven fabric, using biocompatible and biodegradable synthetic fibers, by omitting the final process of cutting a woven 3-dimensional cloth into two, front and back sheets (i.e., a process of cutting through the middle of all the linking strands that link the front and back layers).

As the linker portion of a radiotherapy spacer having the above structure is composed of linker strands extending almost parallel in the thickness direction, the linker strands can be made to fall down easily in the same direction by applying a pressure either in the thickness direction or in a direction intended to induce their falling down, which is considered to explain why the spacer can be easily compressed in its thickness and also shows a great rate of reduction in its thickness. Nevertheless, since such falling down of linker strands does not reach a level that cause their plastic deformation, and also as the linker strands are almost parallel to each other and fall down in concert, no resistance like friction is brought about that would otherwise take place through complex entanglement of adjacent strands. It is considered to explain why the linker strands can freely rise up back to their initial state once the pressure in the thickness direction is released (reconstitution), which directly brings about the recovery of the structure's thickness as a whole, thereby resulting in high reconstitutability.

The radiotherapy spacer according to the present invention can be easily reduced in its thickness by applying a pressure in the thickness direction or in an intended direction to make the linker strands to fall down. The front and back main surface potions are flexible, and the linker portion does not resist curving the spacer, either. Thus, the spacer can be made into the form of a compact roll, by wrapping it around upon itself while sequentially compressing its thickness starting from one of its edges. Likewise, it is also possible to wrap it compact around forceps about to be inserted into a trocar, while sequentially compressing it to reduce its thickness.

Once placed inside the body, just unrolling it with a tool, such as forceps, inserted through the trocar allows the linker strands of the spacer to rise up back to their initial state, thereby recovering the spacer's initial thickness. The spacer thus reconstituted can be inserted between the affected site targeted for radiation and normal tissues around it. In performing this, it is also possible to supplement water (as Ringer's solution or any other body fluid-like medical solution usable at the site) to the spacer as needed. Exuding body fluid or supplemented water permeate the continuous space among numerous linker strands and the main face portions of the spacer, and is retained there to serve as a radiation shield. The present invention thus enables insertion of a spacer with a much greater size than the inner diameter of a trocar into the body of a patient without resorting to open surgery, to shield normal tissues from irradiation.

The radiotherapy spacer according to the present invention is composed of a biocompatible and biodegradable synthetic fibers, which is gradually absorbed after placed in the body and eventually vanishes, without encountering rejection from the body or injuring body tissues. Consequently, with a radiotherapy spacer according to the present invention, there is no need to remove an implanted spacer, therefore no need for additional surgery for its removal. Thus the radiotherapy spacer according to the present invention can greatly contribute to improving the quality of life (QOL) of patients suffering from malignant tumors.

Examples of biocompatible and biodegradable synthetic fibers include, but not are limited to, poly(ester-ether)s, poly(ester carbonate)s, poly(acid anhydride)s, poly(hydroxy alkanoic acid)s, polycarbonate, poly(amide-ester)s, polyacrylates. More specifically, at least one compound can be named that is selected from the group consisting of polyglycolide, poly(L-lactic acid), poly(DL-lactic acid), polyglactin (D/L=9/1), polydioxanone, glycolide/trimethylene carbonate (9/1), polycaprolactone, glycolide-lactide (D, L, DL forms) copolymers, glycolide-$\varepsilon$-caprolactone copolymers, lactide (D, L, DL forms)-$\varepsilon$-caprolactone copolymers, poly(p-dioxanone), and glycolide-lactide (D, L, DL forms)-$\varepsilon$-caprolactone lactide (D, L, DL forms). Among them, particularly preferred ones include polyglycolide, poly(L-lactic acid), and lactic acid/glycolic acid copolymers (with a monomer ratio of lactic acid/glycolic acid not more than 2/8).

The fiber used in producing the radiotherapy spacer according to the present invention may either be monofilament or multifilament. In the case where it is multifilament, it may be in the form of a yarn or an untwisted strand. There is no particular limitation as to the thickness of the strand to be employed, and thus any strand easily available may be used, such as e.g., strand of between 10 dtex and several dozen dtex, though thicker or thinner strand than this may also be used.

Further, there is no particular limitation as to the cross-sectional shape of the monofilament of the strand employed, and it may be any of various shapes, such as circular, triangular, L-shaped, T-shaped, Y-shaped, W-shaped, four-lobed, eight-lobed, flattened, or dog-bone-like, and it may even be a hollow cross section.

The radiotherapy spacer according to the present invention can be prepared in various sizes and shapes suitable to the location and dimensions of the affected site of interest, by cutting a large-surface spacer base material produced as Double Raschel knit or uncut velvet. However, as it is the size and the shape of the main faces that can be adjusted by cutting whereas the thickness is not adjustable, a spacer base material is to be prepared in advance as having a desirable thickness. Although desirable thickness could also vary upon each surgery in a strict sense, the thickness of a spacer base material is preferably in the range of from 5 mm to 20 mm, and more preferably in the range of from 5 mm to 15 mm. Several types spacers that are provided within such size ranges could meet the need in most cases. However, as plural radiotherapy spacers can be used also in such a manner that they are laid upon another, it is also allowed to provide just thin spaces (5 mm thick, for example, within the above ranges) and use them together in an overlapping manner as needed. Thus, while it may be convenient if plural different types of spacers are provided within the above ranges, it is not necessary to so. Also, if implanting spacer thicker than 15 mm is intended, such a need can be met as desired by, e.g., using two or three of 10-mm thick spacers, or two of 15-mm thick spacers.

While there is no clear limitations in particular, the density as a whole of the radiotherapy spacer according to the present invention is preferably in the range of from 50 mg/cm$^3$ to 200 mg/cm$^3$, in general. Density can be adjusted as desired, e.g., by using thicker strands, or kitting or weaving into more condensed lattice, or by employing combination of those methods. Even in the case where the spacer is given an increased density such as 120 mg/cm$^3$, 150 mg/cm$^3$,

or 200 mg/cm$^3$, the whole body of the spacer can be easily compressed into a compact one by, e.g., making densely arranged linker strands fall down with a load applied on an edge of the spacer so as to compress the region under the load, and rolling the spacer while sequentially shifting the position of the load.

In the radiotherapy spacer according to the present invention, it is mainly the linker portion that engages in compression and recovery of its thickness, and for the spacer to have high reconstitutability, the density of the linker portion is preferably 30 mg/cm$^3$ or over. The spacer can be easily compressed in the region where a load is locally applied, and thus can be readily compressed in its entirety by rolling it while sifting the location of the load. Thus, though there is no particular upper limit, it is convenient that the density of the linker portion is within a range of up to 150 mg/cm$^3$, from a practical point of view.

The process for production of a spacer base material may include a sterilization step. A sterilization step may be designed utilizing any of publicly known methods of sterilization, such as autoclave sterilization, EOG sterilization, gamma sterilization, electron beam sterilization, plasma sterilization, and the like.

In the present invention, the terms "rate of compressibility" and "rate of compression resilience" mean the properties determined in accordance with the method stipulated in JIS L 1913 (Test Methods for Nonwovens). The term "rate of compressibility" represents the ratio (%) of reduction in the thickness of a sample relative to its initial thickness, after application of a defined amount of load (pressure) in the thickness direction for a defined length of time, and the term "rate of compression resilience" represents the ratio (%) of recovery of the decrement in thickness that has been caused by compression, within a defined length of time.

The rate of compressibility and the rate of compression resilience can be adjusted by selecting and properly setting such factors as desired as the morphology of the knitted-fabric structure or weave structure forming the main face portions of a spacer; the degree of coarseness or fineness of lattices knitted or woven; the material, thickness, or cross-sectional shape of the strands forming the main face portions and the linker portion; the number of linker stands per unit area of the linker portion (array density: number/cm$^2$); the proportion of the total sum of the area of linker strands' in a cross section parallel to the main faces and passing through the linker portion (area density of linker strands (%)); or the density of the linker portion (mg/cm$^3$).

From the viewpoint of easy compressibility, the rate of compressibility is preferably not less than 70%. And from the viewpoint of high reconstitutability, the rate of compression resilience is preferably not less than 80%.

EXAMPLES

Though the present invention is described in further detail below with reference to an example, it is not intended that the present invention be limited to the example.

(Example)

A sheet of double Raschel knit was produced as a spacer base material using poly(lactic acid) fibers (multifilament of 33 dtex consisting of six twisted monofilaments). The density and the thickness of each sample cut out from the spacer base material are as shown in Table 1. In Table 1, density is expressed as the mean value of samples. Thickness is represented by a measurement $T_0$ obtained under the initial load of 0.5 KPa, as shown below in the part "Physical property measurement".

Fig. 1 schematically shows the general appearance of a radiotherapy spacer (10) prepared by cutting out from the above spacer base material. In Fig. 1, the front and back faces of the radiotherapy spacer (10) (main face portions (11) and (12)) are layers of an identical structure both composed of fibers knitted into a mesh, and the front and back face portions (11) and (12) are linked by a dense collection of numerous, generally parallel linker strands (14) that forms the linker portion (13) between the face portions, into a spacer (10) as an integral structure.

In production of a spacer base material of the example, the number of strands extending in the thickness direction was set at 6720/inch$^2$ (corresponding to array density of 6720/(25.4$\times$10$^{-3}$m)$^2$ = approximately 1040/cm$^2$). With this value and the density of poly(lactic acid) (1.27 g/cm$^3$), the proportion of the total sum of the area of linker strands (14) in unit area of a cross section parallel to the main faces and passing through the linker portion (area density of linker strands) is calculated to be approximately 2.7%. Further, likewise, all the mass in the cross section comes from the linker strands (14), and therefore the density of the linker portion (13) as a whole is calculated to be approximately 34

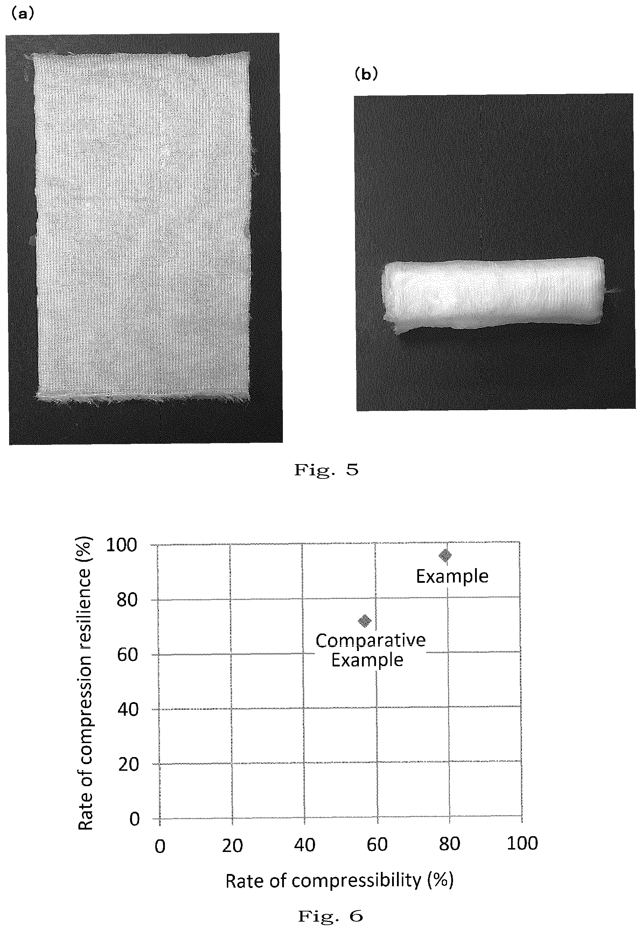

Fig. 5 shows photographs of an radiotherapy spacer (10) (a) in its expanded state before compression, and (b) in its state having been rolled-up into a cylindrical form under simultaneous compressing in the thickness direction, respectively. The radiotherapy spacer (10) according to the present invention can be made into a compact cylindrical form as seen in Fig. 5(b) by rolling it up while compressing it with fingers from an edge onwards.

(Comparative Example)

The same fibers as in the example were cut into short fibers, processed into a fiber sheet by webbing, and into a sheet of nonwoven fabric by needle punching to produce a spacer base material. The density and the thickness of each sample cut out from this spacer base material are as shown in Table 1. In the table, density is shown as the mean value of samples. And thickness is a measurement $T_0$ obtained under the initial load of 0.5 kPa, as shown in "Physical property measurement".

(Physical property measurement)

Five spacers each were cut out from the spacer base materials of Example and Comparative Example, respectively, in the size of 50 mm $\times$ 50 mm to prepare samples, each of which was measured for its rate of compressibility and rate of compression resilience, in accordance with JIS L 1913 (Test Methods for Nonwovens), in the following manner.
Measurement of the rate of compressibility and the rate of compression resilience:

(a) Using a compression resilience testing apparatus, the thickness ($T_0$) of each sample was measured under the initial load of 0.5 kPa.
(b) Then, after application of a load of 30 kPa for one minute, the thickness ($T_1$) of each sample was measured under the same load.
(c) Following removal of the load and being left untouched for one minute, the thickness ($T'_0$) of each sample was measured under the initial load of 0.5 kPa.
(d) The rate of compressibility and the rate of compression resilience was measured using the following formula, and the mean value for the respective 5 samples were determined.

(Formula 1)

$$\text{Rate of Compressbility } P = \frac{T_0 - T_1}{T_0} \times 100$$

(Formula 2)

$$\text{Rate of compression resilience } P_e = \frac{T'_0 - T_1}{T_0 - T_1} \times 100$$

Besides, in addition to the above method, rate of thickness recovery was determined by calculating the ratio of the thickness ($T'_0$) after removal of the load (30 kPa) to the thickness ($T_0$) before compression by the load.

Results:

The rate of compressibility, rate of compression resilience, and the rate of thickness recovery which were determined for each sample based on values of thickness measured, as well as their mean values, are shown in Table 1 below. Further, comparison of results between Example and Comparative Example in the rate of compressibility and the rate of compression resilience is also shown graphically in Fig. 6.

(Table 1.)

Table 1..

| | Mean density (mg/cm³) | Sample No. | $T_0$ (mm) | $T_1$ (mm) | $T'_0$ (mm) | Rate of compressibility (%) | Rate of compression resilience (%) | Rate of thickness recovery (%) |
|---|---|---|---|---|---|---|---|---|
| Example | 76.8 | 1 | 6.44 | 1.34 | 6.19 | 79.193 | 95.098 | 96.1 |
| | | 2 | 6.54 | 1.34 | 6.30 | 79.511 | 95.385 | 96.3 |
| | | 3 | 6.55 | 1.36 | 6.31 | 79.237 | 95.376 | 96.3 |
| | | 4 | 6.54 | 1.34 | 6.37 | 79.511 | 96.731 | 97.4 |
| | | 5 | 6.64 | 1.34 | 6.32 | 79.819 | 93.962 | 95.2 |
| | | Mean value | 6.54 | 1.34 | 6.30 | 79.454 | 95.310 | 96.3 |
| Comparative example | 74.7 | 1 | 4.97 | 2.22 | 4.14 | 55.332 | 69.818 | 83.3 |
| | | 2 | 5.13 | 2.28 | 4.42 | 55.556 | 75.088 | 86.2 |
| | | 3 | 5.20 | 2.21 | 4.37 | 57.500 | 72.241 | 84.0 |
| | | 4 | 4.98 | 2.07 | 4.13 | 58.434 | 70.790 | 82.9 |
| | | 5 | 5.28 | 2.21 | 4.36 | 58.144 | 70.033 | 82.6 |
| | | Mean value | 5.11 | 2.19 | 4.28 | 56.993 | 71.594 | 83.8 |

As evident from Table 1, the spacer of Example according to the present invention exhibited the rate compressibility of about 79.5%. In contrast, the spacer of Comparative Example resisted compression, exhibiting the rate compressibility of only about 57.0% under the same condition. These results mean that while the spacers of Comparative Example were compressed to only about 43.0% of their initial thickness, the spacers of Example were compressed to as much as about 20.5% of their initial thickness, under the same loading conditions, demonstrating a remarkable easy compressibility of the radiotherapy spacer according to the present invention.

Further, when assessed in the rate of compressibility, the spacers of Comparative Example showed, following removal of the load, only about 71.6% recovery of their thickness decrement (about 57.0%) by compression, whereas the spacers of Example of the present invention exhibited about 95.3% recovery of their thickness decrement (about 79.5%). These results indicate a remarkable high reconstitutability of the spacers of Example.

Furthermore, regarding the rate of thickness recovery, the value of 83.3% with the spacers of Comparative Example indicates that their overall thickness reduced by as much as 16% of more through undergoing compression, whereas the corresponding value was 96.3% with the spacer of Example, showing that the decrement of overall thickness of these spacer is only less than 4%.

In addition, as seen in Table 1, in all the values of the rate of compressibility, the rate of compression resilience, and the rate of thickness recovery, the spacers of Example showed notably smaller fluctuation among samples than those with the spacers of Comparative Example, also indicating that the present invention enables production of spacers with greatly increased performance uniformity among products.

Thus, the radiotherapy spacer according to the present invention can be compressed into far more reduced thickness than a spacer consisting of nonwoven fabric having comparable density, and is excellent in the property of thickness recovery after removal of a load, and also superb in the performance uniformity among spacers.

REFERENCE SIGNS LIST

| | |
|---|---|
| 10 | radiotherapy spacer |
| 11 | main face portion |
| 12 | main face portion |
| 13 | linker portion |
| 13z | linker-strand-distributed zone |
| 14 | linker strands |

**Claims**

1. A spacer for radiotherapy that is a flexible structure having front and back main faces and thickness therebetween, and composed of strands of biocompatible and biodegradable synthetic fibers, wherein the spacer consists of

   (a) a pair of main face portions opposing each other with a gap therebetween and respectively defining the main faces of the structure, and
   (b) a linker portion linking between the pair of main face portions ,
   and
   (c) wherein the main face portions comprise a knitted-fabric structure or a weave structure made of the strands, and
   (d) wherein the linker portion is composed as a collection of linker strands formed of the said strands respectively extending in the thickness direction of the spacer and linking the pair of main face portions with each other.

2. The spacer for radiotherapy according to claim 1 possessing a rate of compressibility of at least 70% and a rate of compression resilience of at least 80%, as determined in accordance with JIS L 1913 (Test Methods for Nonwovens).

3. The spacer for radiotherapy according to claim 1 or 2 having a density as a whole of 50-200

4. The spacer for radiotherapy according to one of claims 1-3, wherein the density of the linker portion is at least 30

Fig. 1

Fig. 2

Fig. 3

Fig. 4

(a)

(b)

Fig. 5

Fig. 6

## INTERNATIONAL SEARCH REPORT

| International application No. |
|---|
| PCT/JP2017/033200 |

A. CLASSIFICATION OF SUBJECT MATTER
*A61N5/10*(2006.01)i, *A61L31/06*(2006.01)n

According to International Patent Classification (IPC) or to both national classification and IPC

B. FIELDS SEARCHED

Minimum documentation searched (classification system followed by classification symbols)
A61N5/10, A61L31/06

Documentation searched other than minimum documentation to the extent that such documents are included in the fields searched
Jitsuyo Shinan Koho       1922–1996   Jitsuyo Shinan Toroku Koho   1996–2017
Kokai Jitsuyo Shinan Koho   1971–2017   Toroku Jitsuyo Shinan Koho   1994–2017

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br>Y | WO 2011/055670 A1 (Kobe University),<br>12 May 2011 (12.05.2011),<br>paragraph [0021]<br>& US 2012/0271093 A1<br>paragraph [0032]<br>& EP 2497534 A1          & CN 102596317 A | 1<br>2-4 |
| Y | JP 2013-043066 A (Kawamoto Corp.),<br>04 March 2013 (04.03.2013),<br>paragraph [0013]<br>(Family: none) | 2-4 |
| A | JP 2003-070799 A (Unitika Fibers Ltd.),<br>11 March 2003 (11.03.2003),<br>entire text; all drawings<br>(Family: none) | 1-4 |

☐ Further documents are listed in the continuation of Box C.      ☐ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search<br>27 October 2017 (27.10.17) | Date of mailing of the international search report<br>07 November 2017 (07.11.17) |
|---|---|
| Name and mailing address of the ISA/<br>Japan Patent Office<br>3-4-3,Kasumigaseki,Chiyoda-ku,<br>Tokyo 100-8915,Japan | Authorized officer<br><br>Telephone No. |

Form PCT/ISA/210 (second sheet) (January 2015)

**EP 3 513 837 A1**

### Patent documents cited in the description

- WO 2011055670 A
- WO 2015098904 A